# EUROPEAN PATENT APPLICATION

(11) **EP 1 649 877 A2**
(43) Date of publication of application: **26.04.2006**
(21) Application number: 05255830.1
(22) Date of filing: 21.09.2005
(51) Int. Cl.: A61L 27/30

(54) **Boronised medical implants and process of manufacture**

(30) Priority: 30.09.2004 US 954677
(71) Applicant: DePuy Products, Inc., Warsaw, IN 46581 (US)
(72) Inventor: Smith, Bryan J., Fort Wayne IN 46804 (US); Overholster, Ron, Warsaw, IN 46582 (US); Aust, Sarah, Warsaw, IN 46582 (US)
(74) Representative: Belcher, Simon James

(57) **Abstract**

An orthopaedic joint prosthesis component comprises a metallic body comprising a metal or metal alloy, and a bearing surface disposed on the body, the bearing surface comprising a boronised layer of the metal or metal alloy. A prosthesis component can be made by a method which comprises providing a medical implant or medical implant part having a metallic body, providing a boronising agent which yields boron upon heating, heating the boronising agent to a temperature at which the boronising agent yields boron, contacting at least a portion of the metallic body with the boron produced by the boronising agent, and heating the medical implant or medical implant part to an elevated temperature for a time sufficient for at least a portion of the boron produced by the boronising agent to diffuse into at least a portion of the metallic body of the medical implant or medical implant part.

## Description

This invention pertains to metallic medical implants or medical implant parts comprising a boronised metal layer and processes for producing such medical implants or medical implant parts.

Orthopaedic implants typically must endure significant mechanical stresses and an *in vivo* environment intent on attacking most foreign materials introduced into a patient's body. Therefore, the materials from which such orthopaedic implants are made must combine high strength, corrosion resistance, and tissue compatibility. Furthermore, due to the rigors often associated with revision surgery, it is desirable for the *in vivo* lifespan of an orthopaedic implant to be equal to or exceed the expected remaining lifespan of the recipient of the implant.

One of the variables affecting the longevity of load-bearing orthopaedic implants, such as hip-joint implants, is the rate of wear of the implant's articulating surfaces. A typical hip-joint implant includes a femoral stem, a femoral head attached to the stem, and an acetabular cup against which the femoral head articulates. Wearing of these articulating surfaces generates debris particles that are released into the tissues surrounding the implant. It is generally accepted by orthopaedic surgeons and biomaterials scientists that these debris particles contribute, at least in part, to bone loss at the interface of the orthopaedic implant and the host bone. Indeed, the reaction of the body to these particles includes inflammation and deterioration of the tissues, particularly the bone to which the orthopaedic implant is anchored, through a process known as osteolysis. As the osteolysis progresses, the orthopaedic implant becomes painfully loose and must be revised.

The rate of wear of the articulating surfaces of orthopaedic implants is dependent upon a number of factors. These factors include, but are not limited to, the relative hardness and surface finish of the materials from which the articulating surfaces are made, the coefficient of friction between the materials of the articulating surfaces, the load applied to the articulating surfaces, and the stresses generated at the articulating surfaces. In an effort to decrease the rate of wear of the articulating surfaces of orthopaedic implants, and thereby extend the *in vivo* lifespan of such implants, several attempts have been made to address one or more of the above-identified factors which affect the rate of wear of such articulating surfaces. For example, orthopaedic implants have been developed which are made from relatively hard, wear-resistant, chemically inert ceramics. However, ceramic implants often are brittle and lack the toughness of metallic implants, which can increase the risk of fracture. The brittleness and low toughness of ceramic implants also limits the use of such implants in certain applications, such as the femoral component of a knee arthroplasty. Furthermore, ceramic implants are not compatible with the beaded, porous ingrowth structures used to aid biologic fixation of implants implanted into patients without the use of bone cement.

US-5037438 discloses a prosthetic implant having a coating of blue-black or black zirconium oxide on the bearing surface of the prosthesis body. The relatively thin coatings on the disclosed implant have limited abrasion resistance and may not be suitable for high contact stress applications, such as metal on metal hip bearings. Furthermore, attempts to provide surface layers of zirconium oxide with a thickness greater than approximately 8 to 10 µm have resulted in delamination of the zirconium oxide layer from the zirconium alloy substrate. Such delamination in an implanted prosthesis could lead to significant damage to the prosthesis and require revision surgery.

Other efforts aimed at increasing the wear performance of orthopaedic implants have included ion bombardment of the implant's articulating surfaces (e.g., nitrogen ion implantation), nitriding the implant's articulating surfaces, coating the articulating surfaces with diamond-like carbon or titanium nitride coatings, and oxygen diffusion hardening of, for example, titanium alloy implants. While each of these techniques is capable of producing a hardened articulating surface on the orthopaedic implant, some of the surface coatings or layers produced by such techniques suffer from limited adhesion of the surface coating to the substrate. Furthermore, some of the techniques are only capable of producing very thin coatings or layers of the hardened material, and others produce coatings or layers exhibiting peak hardness vaiues that are not relatively high.

A need therefore exists for orthopaedic implants or implant parts having hardened, wear-resistant articulating surfaces. A need also exists for a process for producing orthopaedic implants or implant parts comprising such hardened, wear resistant articulating surfaces.

The invention provides a medical implant or medical implant part comprising (a) a metallic body comprising a metal or metal alloy, and (b) a bearing surface disposed on the body, the bearing surface comprising a boronised layer of the metal or metal alloy.

The invention also provides a medical implant for implantation into a patient, the medical implant comprising (a) a femoral component for replacing one or more of the patient's femoral condyles, the femoral component having a metallic body comprising a metal or metal alloy and a bearing surface disposed on the body, the bearing surface comprising a boronised layer of the metal or metal alloy, (b) a tibial component for replacing at least a portion of the patient's proximal tibial articular surface, and (c) a polymeric bearing component which rests on the tibial component and confronts the bearing surface of the femoral component.

The invention also provides a medical implant for implantation into a patient, the medical implant comprising (a) a femoral stem for anchoring the implant into the patient's femur, (b) a femoral head which attaches to the upper end of the femoral stem, the femoral head having a metallic body comprising a metal or metal alloy and a bearing surface disposed on the body, the bearing surface comprising a boronised layer of the metal or metal alloy, and (c) an acetabular component for replacing the patient's acetabulum, the acetabular component comprising a liner which confronts the bearing surface of the femoral head.

The invention further provides a process for producing a medical implant or medical implant part, the process comprising the steps of (a) providing a medical implant or medical implant part having a metallic body, (b) providing a boronising agent which yields boron upon heating, (c) heating the boronising agent to a temperature at which the boronising agent yields boron, (d) contacting at least a portion of the metallic body with the boron produced by the boronising agent, and (e) heating the medical implant or medical implant part to an elevated temperature for a time sufficient for at least a portion of the boron produced by the boronising agent to diffuse into at least a portion of the metallic body of the medica implant or medical implant part.

The invention provides a medical implant or medical implant part comprising (a) a metallic body comprising a metal or metal alloy and (b) a bearing surface disposed on the body.

The medical implant or medical implant part can be any suitable metallic medical implant or medical implant part. Suitable implants or implant parts include, but are not limited to, the femoral component (e.g., the component which replaces one or more of the patient's femoral condyles) of an uni-compartmental knee arthroplasty or a total knee arthroplasty, the tibial component (e.g., the component which replaces at least a portion of the patient's proximal tibial articular surface or tibial plateau) of an uni-compartmental knee arthroplasty or a total knee arthroplasty, the femoral head of a hip arthroplasty, the acetabular cup or liner of a hip arthroplasty, the humeral head of a shoulder arthroplasty, the humeral or ulnar component of an elbow arthroplasty, the metacarpal or radial stem of a wrist arthroplasty, the vertebral endplate component of a disc arthroplasty (e.g., a cervical, vertebral disc arthroplasty), and the tibial or talar component of an ankle arthroplasty.

The medical implant or medical implant part of the invention comprises a metallic body. The metallic body of the implant or implant part can comprise, consist essentially of, or consist of any suitable metal or metal alloy (e.g., any metal which readily forms hard borides upon diffusion of boron into the surface at elevated temperatures). For example, the metallic body can comprise a metal or metal alloy selected from the group consisting of cobalt, cobalt alloys, titanium, titanium alloys, and mixtures thereof. Preferably, the metal or metal alloy is selected from the group consisting of cobalt, cobalt-chromium alloys, titanium, titanium-aluminum alloys, and mixtures thereof. Suitable cobalt-chromium alloys include, but are not limited to, the cast, forged, and wrought cobalt-28-chromium-6-molydenum (Co28Cr6Mo) alloys described in, for example, ASTM Standards F75-01, F799-02, and F1537-00, respectively. Suitable titanium-aluminum alloys include, but are not limited to, the titanium-3-aluminum-2.5-vanadium alloy (Ti3A12.5V) described in, for example, ASTM Standard F2146-01 and the titanium-6-aluminum-4-vanadium (Ti6A14V) alloy described in, for example, ASTM Standards F136-02a.

The medical implant or medical implant part comprises a bearing surface disposed on the body. As utilized herein, the term "bearing surface" is used to refer to a portion of the surface of a medical implant or medical implant part which articulately or movably confronts another surface (e.g., the surface of another medical implant or medical implant part) when the medical implant or medical implant part is implanted in a patient. For example, the bearing surface of the medical implant or medical implant part can correspond to the outer surface of a femoral component of a uni-compartmental or total knee arthroplasty, which surface confronts the polymeric bearing component of the arthroplasty. Alternatively, the bearing surface of the medical implant or medical implant part can correspond to the outer surface of the femoral head of a hip arthroplasty, which surface confronts the liner of the acetabular cup.

The bearing surface of the medical implant or medical implant part preferably comprises a boronised layer of the metal or metal alloy from which the body of the implant or implant part is comprised. As utilized herein, the term "boronised" refers to a portion of the metal or metal alloy which comprises boron atoms that have diffused into the metal or metal alloy. For example, the boronised layer can comprise a mixture of borides. Such borides can have, for example, the formula MeB, MeB₂, or Me₂B, wherein Me represents a metal present in the body of the medical implant or medical implant part. For example, when the medical implant or medical implant part comprises Co28Cr6Mo alloy, the boronised layer can comprise a mixture of borides having the formula CoB, Co₂B, as well as other borides of cobalt, chromium, and/or molybdenum. When the medical implant or medical implant part comprises a titanium-aluminum-vanadium alloy (e.g., Ti3A12.5V or Ti6A14V), the boronised layer can comprise a mixture of borides having the formula TiB, TiB₂, as well as other borides of titanium, aluminum, and/or vanadium. Alternatively, the boronised layer can comprise boron atoms that have diffused into the lattice structure of the metal or metal alloy. In such boronised layers, the relatively small boron atoms typically fill a portion of the interstitial spaces (i.e., the spaces between adjacent metal atoms) present in the lattice structure of the metal or metal alloy. As will be understood by those of ordinary skill in the art, the composition of the boronised layer can be different at various points in the boronised layer (e.g., at various depths in the boronised layer). For example, the boronised layer of a medical implant or medical implant part according to the invention can predominantly comprise borides having the formula MeB or MeB₂ in the portion of the boronised layer closest to the surface of the metallic body, while predominantly comprising borides having the formula Me₂B or boron atoms filling a portion of the interstitial spaces in the lattice structure of the metal or metal alloy in the portion of the boronised layer furthest from the surface of the metallic body.

The boronised layer of the metal or metal alloy can have any suitable thickness. Typically, the boronised layer has a thickness of about 1 µm or more (i.e., the boronised layer extends at least about 1 µm below the surface of the metallic body). Preferably, the boronised layer has a thickness of about 2 µm or more, more preferably about 3 µm or more, and most preferably about 5 µm or more (e.g., about 6 µm or more, or about 8 µm or more). The boronised layer typically has a thickness of about 75 µm or less (e.g., about 70 µm or less, about 65 µm or less, about 60 µm or less, about 50 µm or less, or about 40 µm or less). In certain embodiments, the boronised layer preferably has a thickness of about 5 µm to about 30 µm (e.g., about 6 µm to about 30 µm, about 8 µm to about 30 µm, or about 15 µm to about 25 µm).

The boronised layer of the metal or metal alloy typically is harder than the untreated metal or metal alloy (e.g., the metal or metal alloy prior to boronising). Preferably, the boronised layer of the metal or metal alloy has a higher Knoop hardness than the untreated metal or metal alloy. The Knoop hardness of the boronised layer can be determined using any suitable technique. Typically, the Knoop hardness of the boronised layer is determined using the technique described in ASTM Standard E384-99el. In certain embodiments, the boronised layer of the metal or metal alloy has a Knoop hardness of about 1000 HK₅₀ or more, preferably about 1500 HK₅₀ or more, more preferably about 1750 HK₅₀ or more, and most preferably about 2000 HK₅₀ or more (e.g., about 2100 HK₅₀ or more).

In a preferred embodiment, the invention provides a knee arthroplasty implant (e.g., a uni-compartmental knee arthroplasty implant or a total knee arthroplasty implant). In such an embodiment, the medical implant preferably comprises (a) a femoral component for replacing one or more of the patient's femoral condyles, the femoral component having a metallic body comprising a metal or metal alloy and a bearing surface disposed on the body, the bearing surface comprising a boronised layer of the metal or metal alloy, (b) a tibial component for replacing at least a portion of the patient's proximal tibial articular surface, and (c) a polymeric bearing component which rests on the tibial component and confronts the bearing surface of the femoral component. The characteristics of this embodiment of the medical implant or medical implant part of the invention (e.g., the composition of the metallic body, the composition of the boronised layer, the thickness of the boronised layer, the hardness of the boronised layer, etc.) can be the same as those set forth above.

In another preferred embodiment, the invention provides a hip arthroplasty implant. In such an embodiment, the medical implant preferably comprises (a) a femoral stem for anchoring the implant into the patient's femur, (b) a femoral head which attaches to the upper end of the femoral stem, the femoral head having a metallic body comprising a metal or metal alloy and a bearing surface disposed on the body, the bearing surface comprising a boronised layer of the metal or metal alloy, and (c) an acetabular component for replacing the patient's acetabulum, the acetabular component comprising a liner which confronts the bearing surface of the femoral head. The characteristics of this embodiment of the medical implant or medical implant part of the invention (e.g., the composition of the metallic body, the composition of the boronised layer, the thickness of the boronised layer, the hardness of the boronised layer, etc.) can be the same as those set forth above.

The inventive medical implant or medical implant part can be prepared in any suitable manner. For example, the inventive medical implant or medical implant part can be prepared using methods similar to those used to boronise other types of metals, such as the process described in Knotek et al., "Surface Layers on Cobalt Base Alloys by Boron Diffusion," *Thin Solid Films,* 45:331-339 (1977).

The invention further provides a preferred process for producing a medical implant or medical implant part comprising a boronised layer of a metal or metal alloy. In particular, the process comprises the steps of (a) providing a medical implant or medical implant part having a metallic body, (b) providing a boronising agent which yields boron upon heating, (c) heating the boronising agent to a temperature at which the boronising agent yields boron, (d) contacting at least a portion of the metallic body with the boron produced by the boronising agent, and (e) heating the medical implant or medical implant part to an elevated temperature for a time sufficient for at least a portion of the boron produced by the boronising agent to diffuse into at least a portion of the metallic body of the medical implant or medical implant part.

The medical implant or medical implant part to be subjected to the process of the invention can be any suitable metallic medical implant or medical implant part. Suitable implants or implant parts include, but are not limited to, the femoral component (e.g., the component which replaces one or more of the patient's femoral condyles) of an uni-compartmental knee arthroplasty or a total knee arthroplasty, the tibial component (e.g., the component which replaces at least a portion of the patient's proximal tibial articular surface or tibial plateau) of an uni-compartmental knee arthroplasty or a total knee arthroplasty, the femoral head of a hip arthroplasty, the acetabular cup or liner of a hip arthroplasty, the humeral head of a shoulder arthroplasty, the humeral or ulnar component of an elbow arthroplasty, the metacarpal or radial stem of a wrist arthroplasty, the vertebral endplate component of a disc arthroplasty (e.g., a cervical, vertebral disc arthroplasty), and the tibial or talar component of an ankle arthroplasty.

The process of the invention utilizes a boronising agent which yields boron when it is heated. Certain boronising agents can produce elemental boron (e.g., gaseous elemental boron) upon heating. Other boronising agents can produce, upon heating, boron compounds that are capable of releasing one or more boron atoms into the metal or metal alloy when they contact the metallic body at elevated temperatures. Accordingly, the boronising agent comprises a source of boron. The source of boron can be any suitable source which is capable of yielding boron (e.g., boron which is available to diffuse into the metallic body of the medical implant or medical implant part) upon heating. Suitable sources of boron include, but are not limited to, amorphous boron, crystalline boron, boron trifluoride (BF₃), boron trichloride (BCl₃), boron tribromide (BBr₃), diborane (B₂H₆), trimethyl boride ((CH₃)₃B), triethyl boride ((C₂H₅)₃B), boron carbide (B₄C), borax (Na₂B₄O₇.10H₂O or Na₂B₄O₇), metaboric acid (HBO₂), sodium borofluoride (NaBF₄), boric acid anhydride (B₂O₃), ferroboron, metal borides, and combinations thereof

The boronising agent also can comprise other substances that improve the agent's ability to produce boron for the boronising process or improve the handling characteristics of the boronising agent. For example, the boronising agent can comprise any suitable filler, such as, carbon black, silicon carbide, aluminum oxides, magnesium oxides, silicon oxides, silicates, non-boridable metals, and combinations thereof. The boronising agent also can comprise any suitable activator, such as, a fluoroboride (e.g., a tetrafluoroborate). The process of the invention can utilize a commercially available boronising agent, which typically comprises a combination of a boron source, a filler, and an activator. Suitable commercially available boronising agents include, but are not limited to, the boronising agents sold by BorTec GmbH under the trade mark EKABOR.

Any suitable amount of the boronising agent can be used in the inventive process. For example, when the boronising agent is provided in the form of a powder, the medical implant or medical implant part can be packed into an excess of the boronising agent such that the boronising agent contacts only those portions of the metallic body that are to be boronised. The amount of boronising agent used in the process typically provides an amount of boron that exceeds the amount required to produce a boronised layer having the desired thickness.

Any suitable portion of the metallic body of the medical implant or medical implant part can be contacted with the boron produced by the boronising agent. Preferably, the portion of the metallic body that is contacted with the boron produced by the boronising agent corresponds to a bearing surface disposed on the outer surface of the body of the implant or implant part. However, in certain embodiments, substantially all or all of the outer surface of the metallic body can be contacted with the boron produced by the boronising agent.

The process of the invention comprises the step of heating the boronising agent to a temperature at which the boronising agent yields boron. The process of the invention also comprises the step of heating the medical implant or medical implant part to an elevated temperature for a time sufficient for at least a portion of the boron produced by the boronising agent to diffuse into at least a portion of the metallic body of the medical implant or medical implant part. While the boronising agent and the implant or implant part can be heated to different temperatures, the implant or the implant part and the boronising agent typically are heated to substantially the same temperature. For example, when the boronising agent is provided in the form of a liquid, paste, or solid, the boronising agent typically is applied to the surface of the implant or implant part that is to be boronised, and the implant or implant part and the boronising agent are together heated to a temperature at which the boronising agent yields boron. Alternatively, the boronising agent can be heated separately from the medical implant or medical implant part. In such a process, a carrier gas (e.g., an inert carrier gas such as nitrogen) typically is used to transport at least a portion of the boron produced by the boronising agent to the surface of the metallic body so that the boron can diffuse into the metallic body. For example, US-4404045 discloses a process in which the boronising agent is heated separately from the pieces to be boronised.

The medical implant or medical implant part and the boronising agent can be heated to any suitable temperature. As will be understood by those of ordinary skill in the art, the temperatures suitable for the steps of the process will depend, at least in part, on the identity of the metal or metal alloy present in the metallic body and the particular boronising agent used in the process. Preferably, the medical implant or medical implant part and the boronising agent are heated to a temperature of about 500°C or more, more preferably about 550°C or more, and most preferably about 580°C or more. Typically, the implant or implant part and the boronising agent are heated to a temperature that is below the solidus of the metal or metal alloy (i.e., the temperature at which the metal or metal alloy begins to melt and comprises a mixture of solid and liquid phases). Preferably, the medical implant or medical implant part and the boronising agent are heated to a temperature of about 1300°C or less (e.g., about 1250°C or less, or about 1240°C or less).

As will be understood by those of ordinary skill in the art, heating the medical implant or medical implant part to certain temperatures can negatively impact the stability of the metal or metal alloy from which the metallic body of the implant or implant part is comprised. For example, when the medical implant or medical implant part comprises Co28Cr6Mo alloy, heating the implant or implant part to a temperature of about 650°C to about 1170°C can lead to the formation of a multiple-phase solid region in the implant or implant part in which carbides can precipitate as the microstructure ages at the elevated temperature. While the precipitation of such carbides can increase the yield strength and hardness of the alloy, the precipitation also can decrease the ductility and toughness of the alloy, which effects can become significant when the implant or implant part is heated to a temperature of about 800°C to about 1170°C. Such a change in the mechanical properties of the alloy may be acceptable for many applications (e.g., femoral heads), but may pose a concern where the implant or implant part has a relatively thin cross section and experiences cyclic tensile loading (e.g., the femoral component of a knee arthroplasty). Accordingly, in certain embodiments, such as when the metallic body of the medical implant or medical implant part comprises Co28Cr6Mo alloy, the medical implant or medical implant part and the boronising agent preferably are heated to a temperature of about 550°C to about 800°C, more preferably about 650°C to about 800°C. Alternatively, the medical implant or medical implant part and the boronising agent can be heated to a temperature of about 1180°C to about 1250°C, preferably about 1200°C to about 1240°C.

In another embodiment, such as when the metallic body of the medical implant or medical implant part comprises a cobalt-chromium alloy and maintaining the ductility of the cobalt-chromium substrate is of particular concern, the medical implant or medical implant part and the boronising agent can be heated to a temperature of about 800°C to about 1170°C. While such a temperature range may lead to the precipitation of carbides in an implant or implant part comprising a cobalt-chromium alloy, the medical implant or medical implant part can be subjected to further processing to ensure that the ductility and toughness of the implant or implant part are not significantly negatively affected. In one such embodiment, such as when the metallic body of the medical implant or medical implant part comprises a cobalt-chromium alloy (e.g., Co28Cr6Mo), the process preferably further comprises the steps of (f) heating the medical implant or medical implant part to a temperature of about 1200°C to about 1240°C after at least a portion of the boron produced by the boronising agent has diffused into the metallic body of the medical implant or medical implant part, and (g) rapidly cooling the medical implant or medical implant part to a temperature of about 800°C or less. While not wishing to be bound to any particular theory, it is believed that heating the medical implant or medical implant part to a temperature of about 1200°C to about 1240°C will dissolve any carbides that may have precipitated while the implant or implant part was heated to a temperature of about 800°C to about 1170°C. Furthermore, it is believed that rapidly cooling the implant or implant part to a temperature of about 800°C or less will avoid any further significant carbide precipitation in the implant or implant part.

The medical implant or medical implant part and the boronising agent can be heated in any suitable environment. In order to reduce the potential oxidation of the metal or metal alloy contained in the implant or implant part, the implant or implant part and the boronising agent preferably are heated in a vacuum or reduced pressure atmosphere, an inert atmosphere, or a reducing atmosphere. For example, the implant or implant part and the boronising agent can be heated in an inert gaseous atmosphere, such as an atmosphere comprising argon, nitrogen, or any suitable combination of inert gases. Alternatively, the implant or implant part and the boronising agent can be heated in a reducing gaseous atmosphere, such as an atmosphere comprising hydrogen, dissociated ammonia, forming gas (e.g., a gas containing about 5-30% hydrogen and about 70-95% nitrogen), hydrocarbons, a mixtures of at least two of the aforementioned reducing gases, or a mixture of at least one reducing gas with at least one inert gas.

The medical implant or medical implant part is maintained at an elevated temperature for a time sufficient for at least a portion of the boron produced by the boronising agent to diffuse into at least a portion of the metallic body of the implant or implant part. As will be understood by those of ordinary skill in the art, the amount of time necessary for the boron to diffuse into the metallic body of the implant or implant part will depend upon several factors, such as the type of metal or metal alloy present in the body, the temperature(s) to which the boronising agent and the implant or implant part are heated, and the desired thickness of the resulting boronised layer. Typically, the medical implant or implant part is maintained at the elevated temperature (e.g., the temperature to which the implant or implant part and the boronising temperature were heated so that the boronising agent would yield boron to diffuse into the implant or implant part) for about 30 minutes or more, preferably about 60 minutes or more, and more preferably about 120 minutes or more. Typically, the medical implant or implant part is maintained at the elevated temperature for about 720 minutes or less (e.g., about 660 minutes or less), preferably about 600 minutes or less, and more preferably about 540 minutes or less (e.g., about 500 minutes or less, or about 480 minutes or less). However, those of ordinary skill in the art will readily understand that the amount of time necessary to produce a boronised layer having a desired thickness may be longer when relatively low temperatures are used (for example about 850°C or less) or a relatively thick (e.g., about 8 µm or more) boronised layer is desired.

The characteristics of the medical implant or medical implant part produced by the process of the invention (e.g., the composition of the metallic body, the composition of the boronised layer, the thickness of the boronised layer, the hardness of the boronised layer, etc.) can be the same as those set forth above for the medical implant or medical implant part of the invention.

## Claims

1. A medical implant or medical implant part comprising:
(a) a metallic body comprising a metal or metal alloy, and
(b) a bearing surface disposed on the body, the bearing surface comprising a boronised layer of the metal or metal alloy.

2. The medical implant or medical implant part of claim 1, wherein the metallic body comprises a metal or metal alloy selected from the group consisting of cobalt, cobalt alloys, titanium, titanium alloys, and mixtures thereof.

3. The medical implant or medical implant part of claim 2, wherein the metal or metal alloy is selected from the group consisting of cobalt, cobalt-chromium alloys, titanium, titanium-aluminum alloys, and mixtures thereof.

4. The medical implant or medical implant part of claim 3, wherein the cobalt-chromium alloy is Co28Cr6Mo.

5. The medical implant or medical implant part of claim 3, wherein the titanium-aluminum alloy is selected from the group consisting of Ti3A12.5V and Ti6A14V.

6. The medical implant or medical implant part of claim 1, wherein the boronised layer comprises borides having the formula MeB, MeB₂, or Me₂B, wherein Me represents a metal present in the body of the medical implant or medical implant part.

7. A medical implant for implantation into a patient, the medical implant comprising:
(a) a femoral component for replacing one or more of the patient's femoral condyles, the femoral component having a metallic body comprising a metal or metal alloy and a bearing surface disposed on the body, the bearing surface comprising a boronised layer of the metal or metal aiioy,
(b) a tibial component for replacing at least a portion of the patient's proximal tibial articular surface, and
(c) a polymeric bearing component which rests on the tibial component and confronts the bearing surface of the femoral component.

8. The medical implant of claim 7, wherein the metallic body of the femoral component comprises a metal or metal alloy selected from the group consisting of cobalt, cobalt alloys, titanium, titanium alloys, and mixtures thereof.

9. The medical implant of claim 8, wherein the metal or metal alloy is selected from the group consisting of cobalt, cobalt-chromium alloys, titanium, titanium-aluminum alloys, and mixtures thereof.

10. The medical implant of claim 9, wherein the cobalt-chromium alloy is Co28Cr6Mo.

11. The medical implant of claim 9, wherein the titanium-aluminum alloy selected from the group consisting of Ti3A12.5V and Ti6A14V.

12. The medical implant of claim 7, wherein the boronised layer comprises borides having the formula MeB, MeB₂, or Me₂B, wherein Me represents a metal present in the body of the medical implant or medical implant part.

13. A medical implant for implantation into a patient, the medical implant comprising:
(a) a femoral stem for anchoring the implant into the patient's femur,
(b) a femoral head which attaches to the upper end of the femoral stem, the femoral head having a metallic body comprising a metal or metal alloy and a bearing surface disposed on the body, the bearing surface comprising a boronised layer of the metal or metal alloy, and
(c) an acetabular component for replacing the patient's acetabulum, the acetabular component comprising a liner which confronts the bearing surface of the femoral head.

14. The medical implant of claim 13, wherein the metallic body of the femoral head comprises a metal or metal alloy selected from the group consisting of cobalt, cobalt alloys, titanium, titanium alloys, and mixtures thereof.

15. The medical implant of claim 14, wherein the metal or metal alloy is selected from the group consisting of cobalt, cobalt-chromium alloys, titanium, titanium-aluminum alloys, and mixtures thereof.

16. The medical implant of claim 15, wherein the cobalt-chromium alloy is Co28Cr6Mo.

17. The medical implant of claim 15, wherein the titanium-aluminum alloy selected from the group consisting of Ti3A12.5V and Ti6A14V.

18. The medical implant of claim 13, wherein the boronised layer comprises borides having the formula MeB, MeB₂, or Me₂B, wherein Me represents a metal present in the body of the medical implant or medical implant part.

19. The medical implant of claim 13, wherein the liner is comprised of a metal or metal alloy.

20. The medical implant of claim 19, wherein the portion of the liner which confronts the bearing surface of the femoral head comprises a boronised layer of the metal or metal alloy of which the liner is comprised.

21. A process for producing a medical implant or medical implant part, the process comprising the steps of:
(a) providing a medical implant or medical implant part having a metallic body,
(b) providing a boronising agent which yields boron upon heating,
(c) heating the boronising agent to a temperature at which the boronising agent yields boron,
(d) contacting at least a portion of the metallic body with the boron produced by the boronising agent, and
(e) heating the medical implant or medical implant part to an elevated temperature for a time sufficient for at least a portion of the boron produced by the boronising agent to diffuse into at least a portion of the metallic body of the medical implant or medical implant part.

22. The process of claim 21, wherein the metallic body comprises a metal or metal alloy selected from the group consisting of cobalt, cobalt alloys, titanium, titanium alloys, and mixtures thereof.

23. The process of claim 22, wherein the metal or metal alloy is selected from the group consisting of cobalt, cobalt-chromium alloys, titanium, titanium-aluminum alloys, and mixtures thereof.

24. The process of claim 23, wherein the cobalt-chromium alloy is Co28Cr6Mo.

25. The process of claim 23, wherein the titanium-aluminum alloy is selected from the group consisting of Ti3A12.5V and Ti6A14V.

26. The process of claim 21, wherein the medical implant or medical implant part and the boronising agent are heated to a temperature of about 550°C to about 1300°C.

27. The process of claim 24, wherein the medical implant or medical implant part and the boronising agent are heated to a temperature of about 550°C to about 800°C.

28. The process of claim 27, wherein the medical implant or medical implant part and the boronising agent are heated to a temperature of about 650°C to about 800°C.

29. The process of claim 24, wherein the medical implant or medical implant part and the boronising agent are heated to a temperature of about 800°C to about 1170°C.

30. The process of claim 29, wherein the process further comprises the steps of:
(f) heating the medical implant or medical implant part to a temperature of about 1200°C to about 1240°C after at least a portion of the boron produced by the boronising agent has diffused into the metallic body of the medical implant or medical implant part, and
(g) rapidly cooling the medical implant or medical implant part to a temperature of about 800°C or less.

31. The process of claim 24, wherein the medical implant or medical implant part and the boronising agent are heated to a temperature of about 1180°C to about 1250°C.

32. The process of claim 31, wherein the medical implant or medical implant part and the boronising agent are heated to a temperature of about 1200°C to about 1240°C.
